# EUROPÄISCHE PATENTANMELDUNG

(11) **EP 1 865 070 A1**
(43) Veröffentlichungstag der Anmeldung: **12.12.2007**
(21) Anmeldenummer: 06011991.4
(22) Anmeldetag: 10.06.2006
(51) Int. Cl.: C12Q 1/68

(54) **Verfahren zur Markierung von Produkten mit Nukleinsäuren für den Identitäts- und Herkunftsnachweis der Produkte**

(71) Anmelder: Cognis IP Management GmbH, 40589 Düsseldorf (DE); Eurofins Analytik GmbH, 21079 Hamburg (DE)
(72) Erfinder: Melchior, David, Dr., 51465 Bergisch-Gladbach (DE); Pöpping, Bert, Dr., 21107 Hamburg (DE)
(74) Vertreter: Reinhardt, Jürgen

(57) **Zusammenfassung**

Die vorliegende Erfindung betrifft im Allgemeinen das Gebiet der Produktidentifikation. Insbesondere betrifft die vorliegende Erfindung Verfahren zur Markierung von Produkten mit Nukleinsäuren, um die Identität und Herkunft der Produkte festzustellen, sie nachzuverfolgen und ihre Menge bei Verschnitt bzw. Verdünnung durch andere Produkte zu bestimmen. Weiter betrifft die vorliegende Erfindung die Verwendung von Zeichen- und Zeichenfolgen kodierenden Nukleinsäuren zur Markierung von Produkten sowie Produkte, die Nukleinsäuren umfassen, die Zeichen- und Zeichenfolgen kodieren.

SEQUENZPROTOKOLL

<110>  Cognis IP Management GmbH

<120>  Verfahren zur Markierung von Produkten mit Nukleinsäuren für den
       Identitäts- und Herkunftsnachweis der Produkte

<130>  COG-001

<160>  3

<170>  PatentIn version 3.3

<210>  1
<211>  72
<212>  DNA
<213>  Artificial

<220>
<223>  Sequenz des kodierenden Bereichs in pUC-Cognis

<400>  1
gaggttgcgg tgggatatgt ttagggagcg ggagtggaag taatgtctgc aggtgtggtt      60

tctgctgttt ct                                                          72


<210>  2
<211>  16
<212>  DNA
<213>  Artificial

<220>
<223>  Primer: Tn5-1

<400>  2
ggatctcctg tcatct                                                      16


<210>  3
<211>  20
<212>  DNA
<213>  Artificial

<220>
<223>  Primer: pep3-6

<400>  3
cactcttgtc tcttgtcctc                                                  20

## Beschreibung

Die vorliegende Erfindung betrifft im Allgemeinen das Gebiet der Produktidentifikation. Insbesondere betrifft die vorliegende Erfindung Verfahren zur Markierung von Produkten mit Nukleinsäuren, um die Identität und Herkunft der Produkte festzustellen, sie nachzuverfolgen und ihre Menge bei Verschnitt bzw. Verdünnung durch andere Produkte zu bestimmen. Weiter betrifft die vorliegende Erfindung die Verwendung von Zeichen und Zeichenfolgen kodierenden Nukleinsäuren zur Markierung von Produkten sowie Produkte, die Nukleinsäuren umfassen, die Zeichen- und Zeichenfolgen kodieren.

Üblicherweise bereitet es große Schwierigkeiten, ein Produkt, das von einem Hersteller hergestellt wird, von dem selben Produkt, das von einem anderen Hersteller hergestellt wurde, zu unterscheiden. Dies trifft insbesondere zu, wenn die Hersteller denselben Herstellungsprozess verwenden. Dies führt im gewerblichen Bereich zum großen Problem der Produktfälschung, der nicht autorisierten Verteilung und dem nicht genehmigten Verkauf eines Produkts (z.B: Graumarkthandel und Parallelhandel). Damit verbunden ist auch das Problem einer fälschlichen Produkthaftung des Herstellers für Produkte, die zwar von diesem nicht hergestellt und in den Verkehr gebracht wurden, aber nicht von den eigenen Produkten des Herstellers unterschieden werden können, im Fall, dass durch die gefälschten und gegebenenfalls minderwertigen Produkte Dritte zu Schaden kommen.

Auf der ganzen Welt versehen Hersteller ihre Produkte mit einem unterscheidbaren, kennzeichnenden Erscheinungsbild, z.B. durch die Verpackung des Produkts, so dass Verbraucher ihre Produkte von denen der anderen Hersteller unterscheiden können. Hieraus ergibt es sich, dass die Verbraucher das äußere Erscheinungsbild eines Produkts mit besonderen Qualitätsmerkmalen verbinden und, wenn sie mit diesen Qualitätsmerkmalen zufrieden sind, diese Produkte anderen Konkurrenzprodukten vorziehen. Sobald allerdings die Verbraucher ein Produkt schätzen gelernt haben und es den anderen Konkurrenzprodukten vorziehen, ergibt sich verstärkt das Problem der Produktfälschung. Ein gefälschtes Produkt stellt im Wesentlichen ein Produkt dar, das ein Erscheinungsbild oder ein Markenzeichen aufweist, das dem echten Produkt so ähnlich ist, dass die Verbraucher auch das gefälschte Produkt dem ursprünglichen Hersteller zuordnen und entsprechend kaufen. Das Material eines gefälschten Produkts kann dasselbe oder ein anderes Material sein als das, welches für das echte Produkt verwendet wird. Oft ist das Material des gefälschten Produkts dasselbe Material, allerdings von minderer Qualität. Oft ist das Material des Produkts des redlichen Herstellers auch zu einem prozentualen Anteil mit minderwertigem Material vermischt.

Neben der Produktfälschung ist die Produktverdünnung (auch Produktverschnitt oder Produktvermischung genannt) ein weiteres großes Problem redlicher Hersteller. Von einer Produktverdünnung spricht man, wenn ein Produkt mit einem Verdünnungsmittel, gegebenenfalls einem Verdünnungsmittel geringerer Qualität, versetzt wird. Insbesondere tritt dieses Problem bei flüssigen Produkten wie Ölen oder Öl-basierten Produkten auf.

Die oben genannten Probleme der Produktfälschung und Produktverdünnung führen nicht nur zu einem wirtschaftlichen Schaden des Herstellers des echten Produkts, sondern verschlechtern das ursprüngliche Produkt, beziehungsweise es tritt ein minderwertiges Produkt an die Stelle des echten (ursprünglichen) Produkts, so dass die Verbraucher unter Umständen geschädigt werden, was in Folge zur einer massiven Schädigung des Rufes des echten Produkts führt.

Im Rahmen des Wettbewerbs haben Hersteller nicht nur ein Interesse daran, ihre Produkte von den Produkten der Wettbewerber oder Produktfälscher zu unterscheiden, um so die Fälschung oder Verdünnung ihrer Produkte zu verhindern, sondern auch, ihre eigenen Produkte, sobald sie in den Handel gelangt sind, weiterhin einem genauen Herstellungszeitpunkt (d.h. Herstellungstag bzw. Herstellungsdatum) und Herstellungsort zuzuweisen. Üblicherweise geschieht dies durch eine auf dem Produkt angebrachte Artikel- und/oder Chargennummer. Da diese jedoch meist auf der Verpackung des Produkts angebracht ist, ist diese Artikel- und/oder Chargennummer leicht vom Produkt zu entfernen, so dass das Produkt anschließend nicht mehr dem genauen Herstellungszeitpunkt beziehungsweise Herstellungsort zuzuweisen ist.

Daher besteht ein Bedarf an einem Verfahren, Produkte zu markieren, um sie von anderen, gegebenenfalls gefälschten und/oder verdünnten Produkten von Wettbewerbern, zu unterscheiden und sie gleichzeitig jederzeit dem betrieblichen Herstellungsort bzw. Herstellungszeitpunkt oder Charge zuweisen zu können.

Im Stand der Technik wurden bislang lediglich Verfahren vorgeschlagen, die eine Markierung von Produkten ermöglichen, um diese von gefälschten und/oder verdünnten Produkten von Wettbewerbern zu unterscheiden.

EP 1 199 371 A2 beschreibt die Markierung von Produkten mit Markerliganden (z.B. Hormonen, Chemikalien, etc.), die in einem Nachweissystem (z.B. in einer Zelle) an die Ligandenbindedomäne eines Transkriptionsfaktors binden und diesen aktivieren, was zu einer nachweisbaren Expression eines durch diesen Transkriptionsfaktor regulierten Reportergens führt.

In ähnlicher Weise beschreibt EP 0 409 842 B1 die Markierung von Produkten mit einer Markerverbindung (z.B. kleinen organischen Molekülen), die in der Lage ist, an einen komplementären Bindungspartner (z.B. einen Antikörper) zu binden, so dass ein immunologisch nachweisbares Bindungspaar ausgebildet wird. Der Nachweis erfolgt dabei beispielsweise enzymchemisch in einer ELISA-Reaktion oder mittels einer Affinitätschromatographie.

EP 0 327 163 B1 beschreibt die Markierung von Produkten mit einem Antigen als Markermolekül (z.B. ein(e) Protein, Lipid, Nukleinsäure, etc.), das in einem spezifischen Immunoassay identifiziert werden kann. Der Nachweis der entsprechenden Antigen/Antikörper-Reaktion erfolgt beispielsweise in einer ELISA-Reaktion.

WO 95/06249 beschreibt die Markierung von Produkten mit einem Hapten, das gegebenenfalls kovalent an eine polymere Verbindung wie ein Oligonukleotid gebunden ist, und einen immunologischen Nachweis des Haptens beispielsweise durch einen Antikörper.

WO 98/33162 beschreibt die Markierung von Produkten mit Markern, die verschiedenen Stoffklassen angehören können, und den spezifischen Nachweis der Marker je nach Markerbeschaffenheit beispielsweise durch immunologische, physikalische oder chemische Verfahren. Als Beispiel nennt WO 98/33162 auch Nukleinsäuren, die den Produkten beigemischt werden, und die beispielsweise mittels SDS-PAGE, HPLC oder Nukleinsäurehybridisierung mit einer komplementären Nukleinsäure nachgewiesen werden können.

In ähnlicher Weise beschreibt WO 87/06383, Produkte mit einem Marker (z.B. einer Nukleinsäure oder einem Protein) zu markieren und den Marker durch einen komplementär bindenden Bindungspartner (z.B. eine hybridisierende komplementäre Nukleinsäure bzw. Antikörper) spezifisch nachzuweisen.

Obwohl die oben genannten Dokumente die Markierung von kommerziellen Produkten mit makromolekularen Stoffen wie Peptiden, Proteinen oder Nukleinsäuren vorschlagen, um die Produkte von äußerlich identischen Produkten von Wettbewerbern, die diesen makromolekularen Stoff nicht enthalten, unterscheiden zu können, existiert im Stand der Technik kein Verfahren zur fälschungssicheren Markierung von Produkten, das dem Hersteller gleichzeitig ermöglicht, seine Produkte, auch nachdem sie in den Handel gelangt sind, mittels der verwendeten Markierung eindeutig dem entsprechenden betrieblichen Herstellungszeitpunkt bzw. dem betrieblichen Herstellungsort zuzuweisen.

Die im Licht des vorstehend dargestellten Stands der Technik zu lösende Aufgabe bestand somit darin, ein Verfahren bereitzustellen, das eine fälschungssichere Markierung und gleichzeitig eine genaue Bestimmung der betrieblichen Herkunft von Produkten ermöglicht.

Diese Aufgabe wird durch den Gegenstand der vorliegenden Patentansprüche gelöst.

Die vorliegende Erfindung betrifft ein Verfahren zur Markierung eines Produkts zu dessen Identitäts- und Herkunftsnachweis, wobei das Verfahren den Schritt umfasst, das Produkt mit einer Nukleinsäure zu markieren; dadurch gekennzeichnet dass die Nukleinsäure einen kodierenden Bereich aufweist, dessen Sequenz einer Zeichenfolge zugeordnet ist.

Insbesondere betrifft die vorliegende Erfindung die Markierung von Produkten mit Nukleinsäuren, vorzugsweise mit DNA. Dabei weisen die Nukleinsäuren jeweils eine spezifische Nukleotidsequenz auf, die eine Zeichenfolge, vorzugsweise eine alphanumerische Buchstaben- und/oder Ziffernfolge, kodiert, wobei die mittels der Nukleotidsequenz kodierte Zeichenfolge z.B. die Produkt- und/oder Chargennummer des Produkts darstellt und so die exakte Bestimmung der betrieblichen Herkunft und/oder des Herstellungszeitpunkts des Produkts ermöglicht. Weiter betrifft die vorliegende Erfindung die Verwendung von Zeichenfolgen kodierenden Nukleinsäuren zur Markierung von Produkten sowie Produkte, die Zeichenfolgen kodierende Nukleinsäuren umfassen.

Unter dem Ausdruck ,,Markierung eines Produkts", wie er hierin verwendet wird, versteht man die Assoziierung eines Markers mit einem Produkt, so dass dessen Herkunft, Identität, Herstellungszeitpunkt, Herstellungsort, Charge und/oder Haltbarkeit sowie andere technische Informationen über das Produkt durch die Markierung ermittelt werden können. Die Identifizierung eines markierten Produkts kann darüber hinaus Folgendes erleichtern:
i) Überwachung des Herstellungs- oder anderer Prozesse, einschließlich Mischungsprozessen von Bestandteilen des Produkts sowie Verpackungsprozessen des Produkts;
ii) Produktüberwachung zu Sicherheits- oder Logistikprozessen, beispielsweise Kenntlichmachung der nationalen Herkunft des Produkts für Zollzwecke und Kenntlichmachung von durch nationale Gesetzgebung regulierten Substanzen wie Giften und Rauschmitteln;
iii) Nachweis und Überwachung von Verunreinigungen des markierten Produkts, einschließlich des Nachweises von toxischen Stoffen, wie Pestiziden, Herbiziden, Düngemitteln, organischen Verunreinigungen, wie PBT und Dioxine, und anderen Chemikalien.
Die Markierung eines Produkts beinhaltet auch die Assoziierung eines Produkts mit einer bestimmten Konzentration des Markers, so dass der Nachweis einer Produktveränderung durch Verdünnung, Konzentrationsänderungen oder die Zugabe von Fremdstoffen erleichtert wird.

Unter dem Begriff "Produkt", wie er hierin verwendet wird, wird jegliche Art von Produkt verstanden, dessen Markierung zu Zwecken der Identifizierung, des Herkunftsnachweises und/oder der Produktnachverfolgung erwünscht ist. Beispielsweise kann eine solche Markierung eines Produkts nötig sein, um nicht lizenzierte oder illegale Vervielfältigungen des Produkts nachzuweisen oder die Identifizierung eines gefälschten und gegebenenfalls minderwertigen oder schädlichen Produkts im Sinne des Verbraucherschutzes zu ermöglichen. Das Produkt kann in jeder beliebigen physikalischen Form vorliegen. Beispielsweise kann das Produkt ein flüssiger Stoff oder ein Fluid, ein fester Stoff, eine Dispersion, eine Emulsion, ein Latex oder eine halbfeste Matrix sein. Beispiele für feste Produkte sind, ohne darauf beschränkt zu sein, pharmazeutische Formulierungen wie Tabletten, Kapseln oder Puder; feste Formulierungen von Agro-Chemikalien wie Insektizide, Fungizide, Düngemittel und Saatstoffe; Explosivstoffe; Textilien wie Bekleidung; Datenträger wie DVDs, CDs, Kassetten, Floppydisks; Elektrogeräte wie Fernsehgeräte, Computer und Radios; Bestandteile von Fahrzeugen und Kameras; Papier wie Dokumente, vertrauliche Papiere, Notizen; Verpackungsmaterialen; chemische Produkte wie Farbstoffe, Tinten, Gummi; Kosmetika wie Cremes; Nahrungsmittelprodukte; Baumaterialen wie Asphaltzusätze, Dachziegel und Beton. Beispiele für Fluide oder flüssige Produkte sind, ohne darauf beschränkt zu sein, Öl-basierte Produkte wie Gleitöle, hydraulische Öle, Schmierstoffe, Treibstoffe, wie Benzin, Diesel oder Kerosin, Rohpetroleum, und Petroleumprodukte, Farben, Farbzusätze, Plastikzusätze, Adhäsiva, Beschichtungen, Keramiken, Erdöl-basierte Chemikalien wie Polymere, Parfüme und andere Kosmetika, Getränke wie Wasser, Milch, Wein, Whiskey, Sherry, Gin und Wodka und andere alkoholische und nicht alkoholische Getränke, flüssige pharmazeutische Formulierungen wie Sirupe, Emulsionen und Suspensionen, flüssige agro-chemische Formulierungen wie Pestizide, Insektizide und Herbizide, industrielle Lösungsmittel.

Unter dem Begriff "Nukleinsäure", wie er hierin verwendet wird, versteht man Desoxyribonukleinsäure (DNA) oder Ribonukleinsäure (RNA). Die Nukleinsäure kann neben den üblichen Nukleobasen bzw. Ribonukleosiden Adenin/Adenosin (A), Thymin/Thymidin (T), Cytosin/Cytidin (C), Guanin/Guanosin (G) und Uracil/Uridin (U) Nukleobasen- und/oder Ribonukleosid-Analoga, wie Inosin, umfassen. Die Nukleinsäure kann an ihrem 5'- und/oder 3'-Ende Modifikationen aufweisen, die ihre Stabilität erhöhen. Die Länge der für die Markierung von Produkten verwendeten Nukleinsäuren ist vorzugsweise 10 bis 5000 bp; 15 bis 4500 bp, 20 bis 4000 bp, 25 bis 2000 bp, 30 bis 1500 bp, 35 bis 1000 bp, 40 bis 500 bp, 45 bis 250 bp, 50 bis 200 bp, 55 bis 100 bp. Bei der zur Markierung verwendeten Nukleinsäure kann es sich um eine lineare oder zirkuläre Nukleinsäure handeln. Im Fall einer zirkulären Nukleinsäure kann die Nukleinsäure ein Plasmid oder ein Cosmid sein. Die zur Markierung verwendete Nukleinsäure kann auch verpackt vorliegen. Beispielsweise kann die zur Markierung verwendete Nukleinsäure in einer Proteinhülle, beispielsweise als Phage, vorliegen. Die Nukleinsäure kann auch in einem Liposom verpackt vorliegen. Die zur erfindungsgemäßen Markierung eines Produkts verwendete Nukleinsäure kann neben der Sequenz, welche die erwünschte Zeichenfolge kodiert (hier als *"kodierender Bereich"* bezeichnet), weitere Bereiche umfassen, die Sequenzen aufweisen, welche die Amplifizierung und/oder Sequenzierung des kodierenden Bereichs ermöglichen oder erleichtern. Als weitere Bereiche kann die zur Produktmarkierung verwendete Nukleinsäure mindestens zwei Primerhybridisierungsstellen umfassen, von denen mindestens eine in 5'-Richtung (stromaufwärts) des kodierenden Bereichs und mindestens eine in 3'-Richtung (stromabwärts) des kodierenden Bereichs lokalisiert ist. Die Primerhybridisierungsstellen stellen Bereiche der Nukleinsäure dar, die eine Sequenz aufweisen, die zu der jeweiligen Sequenz der für die Nukleinsäure-Amplifikation (Nukleinsäure-Vervielfältigung) oder -Sequenzierung verwendeten Primer (Oligonukleotide) komplementär sind und somit die Bindung (Hybridisierung) der Primer an die Nukleinsäure an den Primerhybridisierungsstellen erlauben. Die Länge der Primerhybridisierungsstellen entspricht der Länge der verwendeten Primer. Die Primerhybridisierungsstellen können direkt an den kodierenden Bereich der Nukleinsäure anschließen oder von diesem durch Spacer getrennt sein. Vorzugsweise weisen die Spacer eine Länge von 10-200 bp, 20-150 bp, 30-100, 50-80 bp auf Die Nukleinsäuresequenz in den jeweiligen Spacer-Bereichen kann beliebig sein oder eine definierte Sequenz, z.B. eine Signalsequenz, sein. Vorzugsweise umfassen die zur Markierung von Produkten verwendeten Nukleinsäuren zwei Spacer, welche die Primerhybridisierungsstellen jeweils in 5'- und 3'-Richtung in einem Abstand von vorzugsweise 10-100 bp, 15-90 bp, 20-80 bp, 20-70 bp, 25-50 bp von dem kodierenden Bereich abtrennen.

Unter dem Begriff "Zeichen", wie er hierin verwendet wird, versteht man beliebige Ziffern, Buchstaben oder Symbole. Die beliebige Aneinanderreihung von Zeichen wird hierin als Zeichenfolge bezeichnet. Eine Zeichenfolge ist beispielsweise eine Zahl oder Buchstabenaneinanderreihung. Eine Zeichenfolge kann auch eine Kombination von mindestens einer Zahl oder Ziffer und mindestens einem Buchstaben oder einer Buchstabenaneinanderreihung sein. Vorzugsweise ist eine Zeichenfolge eine alphanumerische Zeichenfolge, die aus einer Kombination von Buchstaben und Ziffern besteht und der eine Bedeutung, beispielsweise eine Produkt- oder Chargennummer, beigemessen wird.

Unter dem Begriff "PCR" oder "Polymerase-Kettenreaktion", wie er hierin verwendet wird, versteht man eine Enzym- und Primer-basierte DNA-Amplifikation. Dabei werden Primer (Oligonukleotide) verwendet, die spezifisch an Primerhybridisierungsstellen binden, die den kodierenden Bereich auf seiner 5'- bzw. 3'-Seite flankieren.

Unter den Begriffen "quantitative PCR", Real-Time-PCR" oder "Real-Time-Polymerase-Kettenreaktion", wie sie hierin verwendet werden, versteht man eine Enzym- und Primer-basierte DNA-Amplifikation, die eine Quantifizierung (Mengenbestimmung) der in einer Probe enthaltenen Nukleinsäure ermöglicht (Isabel Taverniers *et al.* 2001).

Die hierin verwendeten Ausdrücke "RT-PCR" oder *"Reverse Transcriptase PCR"* oder "RT-Polymerase-Kettenreaktion" bezeichnen ein Verfahren zur Enzym- und Primer-basierten RNA-Amplifikation. Hierzu wird eine bestimmte Boten-RNA (mRNA) zunächst mittels einer reversen Transkriptase in komplementäre DNA (cDNA) umgeschrieben. Anschließend erfolgt eine PCR oder quantitative PCR.

Der hier verwendete Ausdruck "Primer" bezeichnet einzelsträngige DNA-Oligonukleotide, die unter Ausbildung einer DNA-Doppelhelix oder DNA-RNA-Doppelhelix spezifisch an eine einzelsträngige DNA- oder RNA-Matrize hybridisieren und ein freies 3'-OH-Ende besitzen. Diese molekulare Primer-Matrizen-Struktur ist als Startpunkt für eine DNA- oder RNAabhängige DNA-Polymerase geeignet. Die in der PCR verwendeten Primer besitzen eine Länge von 6-50 Basen, wobei sequenzspezifische PCR in der Regel Primer ab ca. 15 Basen erfordern. Bevorzugte Primerlängen sind 6-50, 10-45, 12-40,15-35,15-30,20-45,25-40 Basen.

Ein Aspekt der vorliegenden Erfindung betrifft ein Verfahren zur Markierung eines Produkts mit einer oder mehreren Nukleinsäuren, wobei das Verfahren den Schritt umfasst, ein zu markierendes Produkt mit einer Nukleinsäure in Kontakt zu bringen, wobei die Nukleinsäure eine Nukleinsäuresequenz aufweist, der eine Zeichenfolge zugewiesen ist.

Die zur Markierung eines Produkts verwendete Nukleinsäure kann mit dem Produkt auf eine vielfältige Weise assoziiert werden. Die Nukleinsäure kann in dem gesamten Produkt oder in einem Teil oder Teilen des Produkts vorhanden sein. Die Nukleinsäure kann dabei in dem gesamten Produkt homogen verteilt sein oder nur in einem Teil des Produkts homogen verteilt vorliegen.

In einer Ausführungsform der vorliegenden Erfindung wird die Nukleinsäure mit dem zu markierenden Produkt in Kontakt gebracht, indem die Nukleinsäure dem Produkt direkt beigemischt wird. Wenn das Produkt ein Fluid, ein flüssiges oder halbfestes Produkt oder ein Pulver ist, wird die Nukleinsäure direkt mit dem Produkt vermischt. Wenn es sich bei dem zu markierenden Produkt um einen Feststoff handelt, der während des Produktionsprozesses flüssig ist, wird die Nukleinsäure während des flüssigen Zustands des Produkts dem Produkt beigefügt und liegt nach Verfestigung des Produkts im festen Produkt vor. Alternativ kann die Nukleinsäure auf die Oberfläche des Produkts aufgetragen werden. Verfahren zur Auftragung der Nukleinsäure auf ein festes Produkt umfassen Farbbeschichtungen, Spraybeschichtungen, Aufbürstungen, Aufdampfung, Tauchbeschichtungsverfahren oder mechanische Applikation. Alternativ kann die Nukleinsäure in Ummantelungszusammensetzungen eingebracht werden, die anschließend auf die Oberfläche des zu markierenden Produkts aufgebracht werden. Beispielhafte Ummantelungszusammensetzungen beinhalten Farben, Lacke, Plastik- oder Gummi-basierte Beschichtungen sowie andere Beschichtungen, die im Stand der Technik bekannt sind. Diese Beschichtungen können beispielsweise auf Kreditkarten, Hologramme, Produktverpackungen, Etiketten oder andere visuelle Kennzeichnungen (z.B. Markenzeichen oder Logos) aufgebracht werden. Die nukleinsäurehaltigen Beschichtungen können direkt auf die Oberfläche des zu markierenden Produkts aufgebracht werden.

In einer weiteren Ausführungsform der Erfindung wird die Nukleinsäure in einer Tintenformulierung aufgelöst oder suspendiert und beispielsweise mittels eines Tintenstrahlverfahrens auf das Produkt aufgetragen.

In einer Ausführungsform der vorliegenden Erfindung liegt die zur Markierung des Produkts verwendete Nukleinsäure in einer Konzentration von vorzugsweise 10⁴ bis 10¹⁰ Kopien DNA oder RNA pro Gramm des Produkts, besonders bevorzugt 10⁴ bis 10⁸ Kopien DNA oder RNA pro Gramm des Produkts, vorzugsweise 10⁴ bis 10⁶ Kopien DNA oder RNA pro Gramm des Produkts vor.

Gemäß der vorliegenden Erfindung weist die zur Markierung eines Produkts verwendete Nukleinsäure eine Nukleinsäuresequenz auf, die eine Zeichenfolge, beispielsweise eine alphanumerische Buchstaben- und/oder Ziffernfolge (Zahl) kodiert.

Die Kodierung einer Zahl, insbesondere einer Dezimalzahl, erfolgt allgemein dadurch, dass die zu kodierende Dezimalzahl in das Binär- (2er-), das Tertiär- (3er-) oder Quartär- (4er-)-Zahlensystem überführt wird. Jeweils identische Ziffern der in den verschiedenen Zahlensystemen resultierenden Zahlen werden anschließend je einer Nukleobase bzw. einem Nukleotid zugeordnet. Der kodierende Bereich der zur Markierung des Produkts verwendete Nukleinsäure wird daraufhin synthetisiert, wobei die Nukleotidabfolge der Ziffernfolge der Binär-, Tertiär- oder Quartärsystem-Zahl entspricht.

Die nach Umrechnung der Dezimalzahl resultierende Zahl besteht im Fall des Binärsystems aus den Ziffern 0 und 1, im Fall des Tertiärsystems aus den Ziffern 0, 1 und 2, und im Fall des Quartärsystems aus den Ziffern 0, 1, 2 und 3. Für die Kodierung wird nun jeweils ein in der Nukleotidsequenz verwendetes Nukleotid einer Ziffer zugewiesen. Wenn die Umrechnung ins Binärsystem erfolgt, kann die Ziffer 0 beispielsweise A, T, C oder G zugewiesen werden. Die Ziffer 1 kann dabei ebenfalls einem der Nukleotide A, T, C oder G zugewiesen werden, wobei zu beachten ist, dass beide Ziffern unterschiedlichen Nukleotiden zugewiesen werden. Bei der Verwendung des Tertiär- und Quartärsystems erfolgt die Ziffernzuweisung entsprechend. Dies bedeutet im Fall des Tertiärsystems, dass die Ziffern 0, 1 und 2 jeweils einem der Nukleotide A, T, C oder G zugewiesen werden. Im Fall des Quartärsystems sind alle 4 Nukleotide A, T, G und C einer Ziffer von 0, 1, 2 und 3 zugewiesen. Die Zuordnung erfolgt bei allen verwendeten Zahlensystemen willkürlich. Im Fall, dass neben den Nukleotiden A, T, G und C weitere Nukleotid-Analoga in der Nukleotidsequenz verwendet werden, z.B. Inosin, kann je nach Anzahl der in der Nukleotidsequenz vorhandenen unterschiedlichen Nukleotide eine Umrechnung der Dezimalzahl ins Quintär-(5er)- oder jedes entsprechend höhere Zahlensystem erfolgen.

In einer bevorzugten Ausführungsform wird die zu kodierende Dezimalzahl, z.B. die Produkt- und/oder Chargennummer in das Binärsystem umgewandelt. Dabei entsprechen die Ziffern 0 und 1 je einem der Nukleotide A, T, G oder C im Fall, dass es sich bei der Nukleinsäure DNA handelt. Für den Fall, dass es sich bei der Nukleinsäure um RNA handelt, entsprechen die Ziffern 0 und 1 je einem der Nukleotide A, U, G oder C.

In einer bevorzugten Ausführungsform wird die Ziffer 0 dem Nukleotid A und die Ziffer 1 dem Nukleotid G zugewiesen. Wird beispielsweise die Nummer 33380220 in den Binärzahlenkode umgewandelt, ergibt sich die Ziffernfolge 1111111010101011101111100. Wird der Ziffer 0 das Nukleotid A und der Ziffer 1 das Nukleotid G zugewiesen, ergibt sich daraus die der oben genannten Ziffernfolge entsprechende DNA-Sequenz GGGGGGGAGAGAGAGGGAGGGGAA.

Die Kodierung von Buchstaben einer alphanumerischen Zeichenfolge mittels der Sequenz des kodierenden Bereichs der zur Markierung eines Produkts verwendeten Nukleinsäure erfolgt in Abhängigkeit davon, welches Zahlensystem für die Umwandlung des Zahlenanteils der alphanumerischen Zeichenfolge verwendet wurde. Für die Kodierung der Buchstaben werden dabei die Nukleotide verwendet, die nicht für die Kodierung des Zahlenanteils verwendet werden und daher frei verfügbar sind. Im Fall, dass der Zahlenanteil in das Binärsystem umgewandelt wurde und die kodierende Nukleinsäure ein unmodifiziertes DNA-Molekül ist (d.h. ohne Verwendung von Nukleotid-Analoga synthetisiert wurde), stehen für die Kodierung von Buchstaben die zwei verbleibenden Nukleotide zur Verfügung. Wenn die Ziffern 0 und 1 beispielsweise durch die Nukleotide A und G belegt sind, stehen für die Buchstabenkodierung die verbleibenden Nukleotide C und T zur Verfügung. Je nach Anzahl der für die Kodierung benötigten unterschiedlichen Buchstaben werden die Buchstaben durch Tripletts, Quadrupletts oder Quintupletts kodiert. Unter einem Triplett versteht man die Abfolge von 3 Nukleotiden, unter einem Quadruplett versteht man die Abfolge von 4 Nukleotiden und unter Quintuplett versteht man die Abfolge von 5 Nukleotiden, die als Einheit ein Zeichen, beispielsweise einen Buchstaben; kodieren. Bei Verwendung von Tripletts und für den Fall, dass zwei freie Nukleotide für die Kodierung zur Verfügung stehen, kann maximal eine Kodierung von 8 (=2³) Buchstaben bzw. Zeichen erfolgen, bei der Verwendung eines Quadrupletts kann maximal eine Kodierung von 16 (=2⁴) Buchstaben bzw. Zeichen erfolgen und bei Verwendung eines Quintupletts kann maximal eine Kodierung von 32 (=2⁵) Buchstaben bzw. Zeichen erfolgen.

In einer bevorzugten Ausführungsform der vorliegenden Erfindung erfolgt die Kodierung von Buchstaben unter Verwendung der für die Kodierung zur Verfügung stehenden Nukleotide C und T durch einen Quintuplett-Kode, da mit einem Quintuplett-Kode alle 26 Buchstaben des deutschen Alphabets kodiert werden können. Vorzugsweise werden die Buchstaben A bis Z des deutschen Alphabets durch folgende Quintupletts kodiert:

| | | | | | |
|---|---|---|---|---|---|
| A | CCCCC | B | CCCCT | C | CCCTC |
| D | CCTCC | E | CTCCC | F | TCCCG |
| G | CCCTT | H | CCTTC | I | CTCTC |
| J | TCCTC | K | CCTCT | L | CTTCC |
| M | TCTCC | N | TCCCT | O | TTCCT |
| P | TTCTC | Q | TTTCC | R | CTTTC |
| S | CCTTT | T | CTTTT | U | TTTTT |
| V | TTCCC | W | TCTTC | X | TCCTT |
| Y | CTCTT | Z | TTCCC | | |

Der Fachmann versteht, dass je nach Anzahl der zu kodierenden Zeichen weitere Quintuplett-Kodierungen gebildet und den jeweiligen Zeichen zugewiesen werden können. Der Fachmann versteht auch, dass zur Kodierung der einzelnen Buchstaben oder Zeichen die Einzelnukleotide der Quintuplett auf beliebige andere Weise permutiert werden können, so dass jedem Buchstaben oder Zeichen ein einzigartiges Quintuplett zugeordnet wird.

Nach Zuweisung der Einzelnukleotide zu den einzelnen Ziffern der resultierenden Binär-, Tertiär- oder Quartärsystem-Zahl bzw. der entsprechenden Tripletts, Quadrupletts oder Quintupletts zu den zu kodierenden Buchstaben/Zeichen der alphanumerischen Zeichenfolge erfolgt die Zusammenstellung der kodierenden Nukleotidsequenz und Herstellung der Nukleinsäuremoleküle, welche die kodierende Nukleotidsequenz, d.h. den kodierenden Bereich, aufweisen. Vorzugsweise wird der kodierende Bereich an seinem 5'- und 3'-Ende von Sequenzen flankiert, die zu der Nukleotidsequenz von Primern komplementär sind (Primerhybridisierungsstellen), die für die Amplifikation und/oder Sequenzierung des zwischen den Primern liegenden kodierenden Bereichs verwendet werden. Vorzugsweise sind die Primerhybridisierungsstellen von dem kodierenden Bereich durch Spacer beabstandet. Die Herstellung der Nukleinsäuren, die den kodierenden Bereich und gegebenenfalls Primerhybridisierungsstellen und/oder Spacer umfassen, erfolgt vorzugsweise mittels *in vitro-* Synthese. Die synthetisierte Nukleinsäure kann anschließend mittels molekularbiologischer Standardverfahren in einen Vektor, z.B. ein Plasmid, eingefügt werden.

In einer weiteren bevorzugten Ausführungsform erfolgt die Kodierung von Zeichen, Zahlen/Ziffern und Buchstaben unter Verwendung von Nukleotid-Tripletts. Dabei repräsentiert eine Gruppe von drei Nukleotiden ein Zeichen, eine Zahl/Ziffer oder einen Buchstaben. Besteht die erfindungsgemäße Nukleinsäure aus den Nukleotiden A, T, C und G, kann bei der Verwendung von Tripletts eine Kodierung von maximal 64 (=4³) Zeichen, Zahlen und/oder Buchstaben erfolgen. Die zu kodierenden Zeichen, Zahlen und/oder Buchstaben können den jeweiligen Tripletts beliebig zugewiesen werden. Beispielsweise kann die Zuweisung von Zahlen/Ziffern, Buchstaben und Zeichen wie im Folgenden angegeben erfolgen.

| | | | | | | |
|---|---|---|---|---|---|---|
| caa=0 | | cag = 1 | | cac=2 | cat=3 | cca=4 |
| ccg = 5 | | cct=6 | | cga=7 | cgc=8 | cgg = 9 |
| | | | | | | |
| gaa=a | | gac=b | | gag=c | gat=d | gca=e |
| gcc = f | | gcg = g | | gct = h | gga=i | ggc = j |
| ggt = k | | gta=1 | | gtc = m | gtg=n | gtt=o |
| taa = p | | tac=q | | tag=r | tat=s | tca=t |
| tcc = u | | tcg=v | | tct=w | tga=x | tgc=y |
| tgt=z | | | | | | |
| | | | | | | |
| ata=. | atc=, | | atg=- | att=; | ggg= [Leerstelle] | |

Der Fachmann versteht, dass je nach Anzahl der zu kodierenden Zeichen andere und/oder weitere Tripletts gebildet und den jeweiligen oder weiteren Zeichen zugewiesen werden können.

Gemäß der vorliegenden Erfindung umfasst das erfindungsgemäße Verfahren weiter einen Schritt zum Nachweis der für die Markierung des Produkts verwendeten Nukleinsäure und einen Schritt zur Bestimmung der Sequenz der Nukleinsäure, insbesondere der Sequenz des kodierenden Bereichs. Gemäß dem erfindungsgemäßen Verfahren kann die Nukleinsäure in oder auf dem Produkt nachgewiesen werden, indem das gesamte Produkt, ein Teil des Produkts oder ein Extrakt des Produkts untersucht wird. Die Isolierung der Nukleinsäure kann mit jedem im Stand der Technik bekannten Nukleinsäure-Isolierungsverfahren ausgeführt werden. Wenn es sich bei der zur Markierung des Produkts verwendeten Nukleinsäure um Plasmid-DNA handelt, kann die DNA unter Verwendung des CTAB/Wizard-Isolierungsverfahrens isoliert werden.

In einer spezifischen Ausführungsform der vorliegenden Erfindung wird die Sequenz aus oder von dem Produkt isolierten Nukleinsäure bestimmt. Wenn nur geringe Mengen der Nukleinsäure für die Markierung des Produktes verwendet wurden, wird die isolierte Nukleinsäure vor der Sequenzbestimmung amplifiziert. Die Amplifikation der Nukleinsäure erfolgt gemäß bekannter Nukleinsäureamplifikationsverfahren.

Die Amplifikation der isolierten Nukleinsäure erfolgt, um die Empfindlichkeitsschwelle eines nachgeschalteten physikalisch-chemischen Analysesystems zu überschreiten (Prinzip der selektiven Signalverstärkung). Die am häufigsten genutzte Technologie zur DNA-Amplifikation ist die Polymerasenkettenreaktion (PCR) (US 4,683,195). Bei diesem enzymchemischen Verfahren werden die Nukleinsäuren selektiv unter Verwendung thermostabiler DNAabhängiger DNA-Polymerasen und PCR-Primern vervielfältigt. Gemäß der vorliegenden Erfindung werden Primer verwendet, die zu Sequenzen komplementär sind, die die alphanumerische Zeichenfolge kodierende Sequenz, d.h. den kodierenden Bereich der Nukleinsäure, auf deren 5'- bzw. 3'-Seite flankieren. Durch Kombination von zwei PCR-Primern zu einem PCR-Primerpaar, dessen einzelne Primer jeweils an einen Matrizenstrang der DNA-Zielsequenz und in Bezug auf deren Orientierungen in 3'-Richtung versetzt binden können, erfolgt die exponentielle Amplifikation der dazwischen liegenden DNA-Region. Die PCR ist ein zyklischer Prozess, wobei jeder Zyklus aus mindestens zwei Temperaturschritten, dem Aufschmelzen der doppelsträngigen DNA bei ca. 90-95°C und dem Anlagern sowie dem Verlängern der Primer bei ca. 50-75°C, besteht. Hierfür werden im Allgemeinen Thermocycler verwendet.

Wenn es sich bei der für die Markierung des Produkts verwendeten Nukleinsäure um RNA handelt, wird diese vor dem Amplifikationsschritt durch eine reverse Transkription in cDNA umgeschrieben. Vorzugsweise erfolgt in diesem Fall die reverse Transkription der RNA und anschließende cDNA-Amplinktion mittels einer RT-PCR.

Die Sequenz der aus oder von dem Produkt isolierten und amplifizierten Nukleinsäure wird mittels Sequenzierung ermittelt. Die Sequenzierung erfolgt dabei mit Verfahren, die im Stand der Technik bekannt sind. Beispielhaft seien die Nukleinsäuresequenzierungsverfahren nach Maxam und Gilbert oder Sanger genannt (Sambrook *et al.,* 1989).

Die solchermaßen ermittelte Nukleotidsequenz des kodierenden Bereichs der Nukleinsäure wird dekodiert, um die alphanumerische Produkt- und/oder Chargennummer des Produkts zu ermitteln. Die Dekodierung erfolgt, indem die ermittelte Nukleotidsequenz zunächst in die einzelnen Tripletts, Quadrupletts oder Quintupletts unterteilt wird und diese den vorbestimmten Buchstaben bzw. Zeichen zugewiesen werden. In gleicher Weise werden die Einzelnukleotide ihrer Reihenfolge nach den jeweils vorbestimmten Ziffern des verwendeten Zahlensystems zugewiesen. Abschließend wird die sich ergebende Zahl in die entsprechende Zahl des Dezimalsystems umgewandelt. Zur Dekodierung ist es notwendig zu wissen, welches Nukleotid des kodierenden Bereichs das erste Nukleotid ist, das Teil einer Kodierungseinheit, z.B. eines Tripletts, Quadrupletts oder Quintupletts, ist bzw. welches Nukleotid die erste Ziffer der kodierten Binär-, Tertiär- oder Quartärsystem-Zahl darstellt. Zu diesem Zweck kann einer oder können beide der in der Nukleinsäure vorhandenen Spacer mindestens eine Signalnukleotidsequenz aufweisen, die sich in einem definierten Abstand zur ersten Kodierungseinheit (z.B. Triplett, Quadruplett oder Quintuplett bzw. Einzelnukleotid) im kodierenden Bereich befindet. Beispielsweise kann sich die Signalnukleotidsequenz in dem Spacer befinden, der den kodierenden Bereich in 5'-Richtung "flankiert. Die Signalnukleotidsequenz kann beispielsweise eine Abfolge von GGGGGGGG (Octa-G-Sequenz) sein, die anzeigt, dass sich in einem definierten Abstand vom achten Guanosin-Rest, beispielsweise 20 Nukleotide in dessen 3'-Richtung, das erste Nukleotid befindet, das eine Ziffer kodiert oder Bestandteil einer Kodierungseinheit (z.B. eines Tripletts, Quadrupletts oder Quintupletts) ist.

In einer weiteren Ausführungsform der vorliegenden Erfindung wird die Menge der im - markierten Produkt- vorliegenden Nukleinsäure bestimmt. (Nukleinsäure-Quantifizierung), um festzustellen ob das-Produkt des redlichen Herstellers verschnitten, d.h. verdünnt, wurde. Als Verfahren zur. Quantifizierung von DNA sind beispielhaft zu nennen: UV-Spektrömetrie bei 260 nm, Fluorometrie unter Verwendung von Fluorophoren, die an DNA spezifisch binden, densitometrische Verfahren unter Verwendung von DNA-Referenzfragmenten sowie die quantitative Real-Time-PCR (qPCR). Die Bestimmung der Menge der im erfindungsgemäß markierten Produkt vorliegenden Nukleinsäure erfolgt vorzugsweise mittels einer quantitativen Real-Time-PCR.

In den letzten Jahren machte die PCR Technologie wesentliche Fortschritte durch die Entwicklung schneller Thermocycler und die Etablierung Fluoreszenz-basierter Bestimmung der nach jedem Amplifikationszyklus generierten doppelsträngigen DNA-Fragmente, was die Quantifizierung durch *"rapid-cycle real-time* PCR"-Analysen ermöglicht (z.B. *LightCycler^{®},* Roche Diagnostics Corp.; ABI Prism^{®} 7000 SDS, Applied Biosystems, Darmstadt, Deutschland). Die sensitive Quantifizierung beruht dabei auf dem Nachweis ansteigender Fluoreszenz während der exponentiellen Phase der PCR im Verhältnis zur ursprünglich für die Reaktion eingesetzten DNA-Menge. Die Quantifizierung beruht dabei insbesondere auf der Ermittlung des *"cycle threshold"* (ct)-Werts, der ersten PCR-Reaktionsrunde mit nachweisbarer Fluoreszenz. Unter Zuhilfenahme externer DNA-Standards, deren Konzentration bekannt ist, kann dabei eine absolute Quantifizierung erfolgen. Im Allgemeinen kann der Nachweis der Fluoreszenz sequenzspezifisch durch Hybridisierungssonden oder TaqMan®-Sonden (basierend auf der 5'→3-Exonuklease-Aktivität der *Taq* DNA Polymerase; Roche Diagnostics und Applied Biosystems, Darmstadt, Deutschland) stattfinden, oder sequenzunspezifisch durch selektiv an doppelsträngige DNA bindende Farbstoffe (insbesondere SybrGreen, Invitrogen Inc.).

Die Ermittlung, ob das Produkt des redlichen Herstellers verdünnt wurde, setzt voraus, dass jedes Produkt bzw. jede Produktcharge eine vorbestimmte Menge der für die Markierung verwendeten Nukleinsäure enthält. Vorzugsweise liegt die Nukleinsäure in einer Menge von 10⁴ bis 10¹⁰ Kopien DNA oder RNA pro Gramm des Produkts, vorzugsweise 10⁴ bis 10⁸ Kopien DNA oder RNA pro Gramm des Produkts, vorzugsweise 10⁴ bis 10⁶ Kopien DNA oder RNA pro Gramm des Produkts vor. Die vorbestimmte, bei jedem Produkt bzw. jeder Produktcharge zugegebene; Nukleinsäuremenge pro Gramm des Produkts wird als 100% definiert und kennzeichnet das Produkt als ursprüngliches, d.h. echtes und unverdünntes, Produkt. Von jedem erfindungsgemäß markierten Produkt bzw. jeder erfindungsgemäß markierten Produktcharge wird mindestens eine Rückstellprobe entnommen und aufbewahrt. Nach Isolierung der DNA aus der Rückstellprobe, wird mittels einer Real-Time-PCR die DNA-Menge pro Gramm in der entnommenen Rückstellprobe bestimmt. Der hierbei ermittelte ct-Wert wird als 100% definiert. Zur Erstellung einer Kalibrierungskurve werden Verdünnungen der aus der Rückstellprobe isolierten DNA hergestellt (z. B. eine 10 %-ige, 20 %-ige, 30 %-ige, 40 %-ige, 50 %-ige, 60 %- ige, 70 %-ige oder 80 %-ige Verdünnung) und mittels Real-Time-PCR die jeweiligen ct-Werte ermittelt. Die Prüfung eines Produkts auf seine Echtheit bzw. Unverdünntheit erfolgt, indem die zur Markierung verwendete Nukleinsäure, d.h. die DNA, aus dem Produkt isoliert und mittels einer Real-Time-PCR die DNA-Menge pro Gramm des zu testenden Produkts bestimmt wird. Der dabei ermittelte ct-Wert des zu testenden Produkts wird mit den Kalibrierungs-ct-Werten, die aus den Verdünnungen der aus der entsprechenden Rückstellprobe gewonnenen DNA ermittelt wurden, verglichen. Wurde beispielsweise bei der 50 %-igen DNA-Verdünnung der Rückstellprobe ein ct-Wert von 31 ermittelt und ergibt die DNA-Quantifizierung des auf Echtheit zu testenden entsprechenden Produkts ebenfalls einen ct-Wert von 31, kann daraus geschlossen werden, dass das gestestete Produkt zu 50 % mit anderen Materialien verschnitten, d.h. verdünnt, wurde.

Ein weiterer Aspekt der vorliegenden Erfindung ist die Verwendung einer eine Zeichenfolge, z.B. Produkt- oder Chargennummer, kodierenden Nukleinsäure für die fälschungssichere Markierung eines Produkts.

Ein weiterer Aspekt der vorliegenden Erfindung ist ein Produkt, das mit Nukleinsäuren markiert ist, die eine Nukleinsäuresequenz aufweisen, die eine Zeichenfolge, z.B. eine Wort- bzw. Buchstabenfolge, kodieren.

### BEISPIEL

Die Zeichenfolge (hier: Wort- bzw. Buchstabenfolge) *"Cognis Original - We Know How"* wurde mittels des folgenden Triplett-Codes in eine DNA-Sequenz kodiert.

| | | | | | | |
|---|---|---|---|---|---|---|
| gaa=a | | gac=b | | gag=c | gat=d | gca=e |
| gcc=f | | gcg=g | | gct=h | gga=i | ggc=j |
| ggt=k | | gta=1 | | gtc=m | gtg=n | gtt=0 |
| taa=p | | tac=q | | tag*=*r | tat=s | tca=t |
| tcc=u | | tcg=v | | tct=w | tga=x | tgc=y |
| tgt=z | | | | | | |
| | | | | | | |
| ata=. | atc=, | | atg=- | att=; | | |

Dabei entspricht jeder Buchstabe bzw. jedes Zeichen der oben genannten Zeichenfolge einem spezifischen Triplett. Für den oben dargestellten Triplett-Code entspricht die Zeichenfolge *,,Cognis Original-We Know How"* die Nukleotidsequenz 5'-gaggttgcggtgggatatgtttagggagcgggagtggaagtaatgtctgcaggtgtggtttctgctgtttct-3' (SEQ ID NO:1). Es wurde ein Nukleinsäuremolekül, das die dargestellte Nukleotidsequenz aufweist, synthetisiert. Die Synthese des Nukleinsäuremoleküls erfolgte durch ein externes Dienstleistungslabor. Das synthetisierte Nukleinsäuremolekül wurde mittels molekulargenetischer Standardverfahren (Sambrook *et al.* (1989)) als kodierender Bereich in die multiple Klonierungsstelle des Plasmids pUC19 mittels gängiger Klonierungsverfahren eingefügt. Das so generierte Plasmid pUC19-Cognis wurde verschieden Matrices zugemischt. Als Bespiel sei hier eine PIT-Emulsion (Emulglade CM SE-PF), ein Spreitöl (Certiol OE) und ein anionisches Tensid (Texapon N70) genannt. Die Matrices wurden von Cognis GmbH&Co KG, Düsseldorf (Deutschland) erhalten. Die den jeweiligen Matrices in verschiedenen Versuchsansätzen zugemischten Nukleinsäuremengen waren jeweils 10³, 10⁴, 10⁵, und 10⁶ Plasmidmoleküle/g Matrix. Die Plasmide wurden homogen in den Matrices verteilt und anschließend mittels des CTBA Wizard DNA-Isolierungsverfahrens (http://gmo-crl.jrc.it/summaries/TC1507-DNAextrc.pdf) wieder aus den Matrices isoliert. Die solchermaßen isolierten Nukleinsäuremoleküle wurden mittels der Primer Tn5-1 : 5'-GGA TCT CCT GTC ATC T-3' (SEQ ID NO:2) und pep3-6: 5'-CAC TCT TGT CTC TTG TCC TC-3' (SEQ ID NO:3) mittels Standard-PCR (Sambrook *et al.* (1989)) amplifiziert. Dabei wurde bei allen getesteten Matrices ab einer ursprünglich zur Markierung eingesetzten Plasmidmenge von 10⁴ Plasmidmolekülen pro Gramm Matrix ein spezifisches DNA-Amplifikat erhalten. Nachdem durch die DNA-Amplifikation die Integrität der für die Markierung der Matrices verwendeten Nukleinsäure pUC19-Cognis bestätigt wurde, wurde das spezifische DNA-Amplifikat, das den kodierenden Bereich umfasst, unter Verwendung molekulargenetischer Standardverfahren (Sambrook *et al.* (1989)) sequenziert. Die ermittelte Nukleotidsequenz des in der multiplen Klonierungsstelle des pUC-Vektors vorliegenden kodierenden Bereichs war 5'-gaggttgcggtgggatatgtttagggagcgggagtggaagtaatgtctgcaggtgtggtttctgctgtttct-3'. Diese ist mit der Sequenz des synthetisierten kodierenden Bereichs identisch (siehe SEQ ID NO:1). Durch Zuweisung der einzelnen Tripletts zu den entsprechenden Zeichen wurde die DNA-Sequenz dekodiert und ergab die ursprüngliche Zeichenfolge,, *Cognis Original-We Know How".*

### Literatur

Isabel Taverniers, Pieter Windels, Erik Van Bockstaele, Marc De Loose (2001). Use of cloned DNA fragments for event-specific quantification of genetically modified organisms in pure and mixed food products, Eur Food Res Technol 213:417-424
Sambrook J, Fritsch EF, Maniatis T (1989). Molecular cloning. A laboratory manual. 2. Auflage, Cold Spring Harbor Laboratory Press, Cold Spring Harbor, USA.
WO 87/06383
WO 95/06249
WO 98/33162
EP 1 199 371 A2
EP 0 409 842 B1
EP 0 327 163 B1
US 4,683,195

## Patentansprüche

1. Verfahren zur Markierung eines Produkts zu dessen Identitäts- und Herkunftsnachweis, wobei das Verfahren den Schritt umfasst, das Produkt mit einer Nukleinsäure zu markieren, **dadurch gekennzeichnet dass** die Nukleinsäure einen kodierenden Bereich aufweist, dessen Sequenz einer Zeichenfolge zugeordnet ist.

2. Verfahren gemäß Anspruch 1, wobei die Zeichenfolge eine Buchstaben- und/oder Ziffernfolge ist.

3. Verfahren gemäß Anspruch 2, wobei die Ziffernfolge eine Dezimalzahl ist.

4. Verfahren gemäß Anspruch 3, wobei die Kodierung der Dezimalzahl in der Sequenz des kodierenden Bereichs **dadurch** erfolgt, dass die Dezimalzahl in ein anderes Zahlensystem umgeformt wird und identische Ziffern der resultierenden Zahl des anderen Zahlensystems einem Nukleotid der zu Nukleinsäure zugewiesen werden.

5. Verfahren gemäß Anspruch 4, wobei die Umrechnung der Dezimalzahl in das Binär-, Tertiär oder Quartärzahlensystem erfolgt.

6. Verfahren gemäß Anspruch 2, wobei die Kodierung der Buchstaben **dadurch** erfolgt, dass jedem Buchstaben ein einzigartiges Triplett, Quadruplett oder Quintuplett von Nukleotiden der zu synthetisierenden Nukleinsäure zugewiesen wird.

7. Verfahren gemäß Anspruch 1, wobei jedem Zeichen der Zeichenfolge ein Triplett von Nukleotiden im kodierenden Bereich der Nukleinsäure zugewiesen wird.

8. Verfahren gemäß einem der vorhergehenden Ansprüche, wobei die Nukleinsäure weiter Primerhybridisierungsstellen und/oder Spacer aufweist.

9. Verfahren gemäß einem der vorhergehenden Ansprüche, wobei die Nukleinsäure eine DNA oder RNA ist.

10. Verfahren gemäß einem der vorhergehenden Ansprüche, wobei die Zeichenfolge die Produktnummer, die Chargennummer, den Herstellungszeitpunkt und/oder den Herstellungsort des Produkts bezeichnet.

11. Verfahren zur Identitäts- und Herkunftsbestimmung eines nach dem Verfahren gemäß einem der Ansprüche 1 bis 10 markierten Produkts, wobei das Verfahren die folgenden Schritte umfasst:
a) Isolieren der zur Markierung verwendeten Nukleinsäure aus dem Produkt,
b) Amplifizieren der isolierten Nukleinsäure
c) Bestimmen der Sequenz der Nukleinsäure und
d) Dekodieren der Nukleinsäuresequenz des kodierenden Bereichs der Nukleinsäure.

12. Verfahren gemäß Anspruch 11, wobei das Amplifizieren der Nukleinsäure mittels PCR erfolgt.

13. Verfahren gemäß Anspruch 11, wobei das Dekodieren **dadurch** erfolgt, dass jedes Nukleotid des kodierenden Bereichs der Nukleinsäure der zuvor bestimmten Ziffer des verwendeten Zahlensystems zugewiesen wird und die resultierende Zahl des verwendeten Zahlensystems in die entsprechende Dezimalzahl umgeformt wird.

14. Verfahren gemäß Anspruch 11, wobei das Dekodieren **dadurch** erfolgt, dass jedes einzigartige Triplett, Quadruplett oder Quintuplett von Nukleotiden des kodierenden Bereichs der Nukleinsäure dem zuvor bestimmten Buchstaben zugewiesen wird.

15. Verfahren gemäß Anspruch 11, wobei das Dekodieren **dadurch** erfolgt, dass jedes einzigartige Triplett von Nukleotiden des kodierenden Bereichs der Nukleinsäure dem zuvor bestimmten Zeichen zugewiesen wird.
